# EUROPEAN PATENT SPECIFICATION

(11) **EP 1 522 312 B1**
(45) Date of publication and mention of the grant of the patent: **21.03.2007**
(21) Application number: 04380188.5
(22) Date of filing: 24.09.2004
(51) Int. Cl.: A61K 38/37

(54) **Biologically stable liquid composition of FVIII, of vWF or of FVIII/vWF complex of human origin.**
Biologisch stabile flüssige Zusammensetzung von FVIII, vWF oder FVIII/vWF-Komplex menschlichen Ursprungs
Compositions biologiquement stables de FVIII, de vWF ou d'un complexe FVIII/vWF d'origine humaine

(30) Priority: 03.10.2003 ES 200302298
(43) Date of publication of application: 13.04.2005
(73) Proprietor: Grifols, S.A., 08005 Barcelona (ES)
(72) Inventor: Grancha Gamon, Salvador, 08400 Granollers (Barcelona) (ES); Jorquera Nieto, Juan Ignacio, 08480 Ametlla del Valles (Barcelona) (ES); Ristol Debart, Pere, Sabadell (ES); Massot Riera, Marta, 08012 Barcelona (ES)
(74) Representative: Durán Moya, Luis-Alfonso

(56) References cited:
- US-A- 5 679 776
- US-B1- 6 320 029

## Description

The present invention relates to a liquid composition in which the stability of the biological activity of Factor VIII (FVIII) or of von Willebrand factor (vWF) or of the Factor VIII/von Willebrand factor complex (FVIII/vWF) is maintained, enabling it to be used therapeutically.

FVIII (coagulation Factor VIII) is a plasma protein essential for the course of the intrinsic pathway of blood coagulation. A deficiency or defect in this FVIII leads to a deterioration in the coagulation mechanism known as haemophilia A, a hereditary disease linked to chromosome X. Von Willebrand factor (vWF) is a plasma protein responsible for platelet adhesion to damaged vascular surfaces and therefore quantitative or qualitative defects of vWF (von Willebrand's disease) produce a deterioration in primary haemostasis. In addition, vWF acts as an FVIII transporter in plasma. (Physiological and clinical aspects of Von Willebrand factor; Haematologica Vol 86, suppl. 4, November 2001)

The FVIII/vWF complex in the natural state has a ratio of 1:100 between FVIII:vWF, that is, there is one molecule of FVIII for approximately every 100 molecules of vWF and therefore the stability results of the FVIII/vWF complex are valid for purified vWF concentrates.

Purified FVIII concentrates are used in clinical practice for the treatment of haemophilia A. FVIII concentrates having a high content of vWF can also be used therapeutically for the treatment of von Willebrand's disease.

The stability of proteins, especially those having a labile biological activity, such as FVIII, is a major problem for therapeutic preparations thereof. This problem has hitherto been solved by lyophilising the product, and therefore therapeutic preparations of the concentrates of FVIII, vWF or FVIII/vWF are available in a lyophilised form in order to preserve the biological activity thereof. At present, no therapeutic concentrate of FVIII, vWF or FVIII/vWF in a liquid final formulation is marketed anywhere in the world (W. Wang et al./International Journal of Pharmaceutics 259/ 2003; 1-15).

Lyophilisation is a process which is expensive and which, in addition, reduces the yield of the product. Accordingly, a liquid formulation would permit greater ease of administration because it would avoid the reconstitution of the lyophilisate. The possibility of making accessible to patients a liquid product (ready for use), already metered into a suitable injecting device, may also bring a psychological benefit in respect of the perception which the patient has of his disease and his dependence on the product.

As above stated, despite the references given hereinafter, there is at present no therapeutic concentrate of FVIII, vWF or FVIII/vWF complex marketed anywhere in the world in a liquid formulation.

There are previous references regarding the stabilisation of FVIII:
- Spanish patent ES 2.111.579 relates to the formulation of an FVIII with arginine and a detergent and/or an organic polymer. This formulation does not foresee the addition of albumin as a stabiliser, achieving therewith a specific activity greater than 1000 IU/mg. This FVIII is in a lyophilised final form.
- US patent 5,399,670 which refers to the formulation with arginine with the aim of facilitating the reconstitution of the lyophilised FVIII concentrate.
- Spanish patent 2.097.120 which relates to the use of carbohydrates in order to stabilise FVIII during the process steps. This FVIII is also in a lyophilised final form.
- US patent 5,925,738 relates to the stable liquid formulation of plasma proteins, especially coagulation factors and specifically FVIII and FIX, although it refers also to vWF. In general, the aim is stability between 4°C and 37°C of up to three years, maintaining 50% of the activity. The majority of the examples refer to FIX, the stability of which is not comparable with that of the FVIII/vWF complex because different molecules are involved. The tests carried out with FVIII are performed at 37°C since the object thereof is not the stability of the final product but its use in pumps for continuous short-term infusion (hours or days). In those tests, the aqueous formulation of FVIII after five days has lost more than 50% of its activity. Therefore, the liquid formulation (in water) does not provide sufficient stability for it to be marketed as a therapeutic product, which requires a shelf life of more than 6 months or preferably more than 1 year.
- PCT WO 96/30041 refers to the stabilisation of r-VIII SQ and FIX in solution. This stabilisation is carried out by the addition of a carbohydrate and the reduction of the oxygen content of the solution or the addition of an antioxidant and/or storage in an atmosphere poor in oxygen or in an inert gas. This formulation has been developed and patented with reference to r-VIII SQ, which is a protein derived by genetic engineering in which a major part of the sequence derived from the corresponding gene has been eliminated. The data adduced demonstrate stability for 12 months at 25°C and for 18 months at 7°C. Because r-VIII SQ is a genetically modified molecule, those results would not be comparable with a natural human FVIII of plasmatic origin or with the FVIII/vWF complex. r-VIII SQ is synthesised "in vitro" in non-human cells whereas human FVIII of plasmatic origin is synthesised "in vivo" in the human liver. This means that significant differences exist between those molecules, such as changes in the content of sugars in the molecule, which are reflected in pharmacokinetic differences, such as the plasma half-life. In addition, r-FVIII SQ includes the deletion of part (almost 40%) of the molecule. Some authors have reported a greater incidence in the development of inhibitors with the use of Factors VIII of recombinant origin. This differentiates these recombinant factors, such as r-VIII SQ, more, if possible, from natural human FVIII. Therefore, it must be considered that the molecule of human FVIII of plasmatic origin is a different molecule from r-VIII SQ and that, because they are different molecules, their stability cannot be compared. It must also be borne in mind that vWF is absent from r-VIII SQ.
- EP 710 114 relates to the formulation of r-VIII SQ, at a minimum concentration of 1000 IU/ml, for its subcutaneous, intramuscular or intradermal administration. The same molecule as in the previous case is involved and therefore the results are not comparable with FVIII, vWF or FVIII/vWF complex of human origin.
- PCT WO 01/03726 (EP 1 194 161) links the presence of specific concentrations of divalent metal ions to an improvement in the stability of r-VIII SQ in solution, specifically Zn²⁺ and CU²⁺, also taking account of the presence of a surfactant (Tween) and histidine. This patent represents another attempt to stabilise r-VIII SQ, which deviates from the subject-matter of the present invention.

There is therefore no prior art on the stabilisation of natural human FVIII, vWF or FVIII/vWF complex, of plasmatic origin, which permits the preservation of the biological activity in a liquid formulation for a period of time sufficient to permit the use thereof as a therapeutic product, that is to say, which is stable in solution for more than 6 months.

The object of the present invention is to provide a liquid formulation which permits sufficient stabilisation of the activity of FVIII, vWF or FVIII/vWF complex (minimum recovery of FVIII and vWF of approximately 50%) for a period of time sufficient to enable it to be used therapeutically (more than 6 months at 5°C).

Investigations carried out by the inventors have brought to light the negative effect of a powerful antioxidant, such as NAC (N-acetyl cysteine), on the activity of vWF and the fact that the replacement thereof by a chelating agent for metals preserves the activity of vWF and also maintains the protective effect on FVIII.

In addition, the investigations carried out have shown that, with the addition of a protease inhibitor (such as antithrombin) and a specific ligand (heparin), an improvement in the stability of the FVIII/vWF complex is achieved.

Therefore it has been discovered that, on a base formulation containing, inter alia: albumin, amino acids, surfactants, antioxidants, such as vitamin C and/or stored in an atmosphere poor in oxygen; by the addition of a chelating agent for metals, such as EDTA (2-100 mmol/l), a protease inhibitor, such as a serine protease inhibitor (serpin) and in particular antithrombin (0.01-1 IU/IU FVIII), and a specific ligand, such as heparin (0.1-10 U/ml), sufficient stability of the FVIII/vWF complex in a liquid formulation is achieved to permit its use as a therapeutic product.

Thus, the present invention provides a [insert claim 1].

### EXAMPLES

FVIII coagulant (FVIII:C) is expressed in International Units and its concentration in International Units/millilitre (IU FVIII/ml). The vWF activity is expressed as ristocetin cofactor (RCo), IU/ml.

### Example 1:

Stability results (percentage recovery of activity), at 5 and 25°C, of an FVIII/vWF complex (25 and 100 IU FVIII/ml) formulated with: albumin 5%, arginine 200 mmol/l, histidine 25 mmol/l, Cl₂Ca 5 mmol/l, at two different concentrations (25 IU/ml and 100 IU/ml).

| 25 IU FVIII/ml | | | | | | |
|---|---|---|---|---|---|---|
| 5°C | | | | | | |
| Time (months) | 1 | 3 | 4 | 6 | 8 | Average slope |
| Recovery of FVIII activity | 94% | 83% | -- | 60% | -- | -6.64% per month |

| 25°C | | | | | | |
|---|---|---|---|---|---|---|
| Time (weeks) | 1 | 3 | 4 | 6 | 8 | Average slope |
| Recovery of FVIII activity | 97% | 79% | 74% | -- | 46% | -6.94% per week |

| 100 IU FVIII/ml | | | | | | |
|---|---|---|---|---|---|---|
| 5°C | | | | | | |
| Time (months) | 1 | 3 | 4 | 6 | 8 | Average slope |
| Recovery of FVIII activity | 98% | 80% | -- | 64% | -- | -6.33% per month |

| 25°C | | | | | | |
|---|---|---|---|---|---|---|
| Time (weeks) | 1 | 3 | 4 | 6 | 8 | Average slope |
| Recovery of FVIII activity | 92% | 83% | 71% | -- | 28% | -8.93% per week |

The results show a good correlation between the FVIII recoveries obtained at 5°C and 25°C, observing in the Table that a period of preservation of one week at 25°C (condition of accelerated stability study) is equivalent to a period of preservation of one month at 5°C (target temperature).

### Example 2:

An FVIII/vWF complex (25 IU FVIII/ml) formulated with:

| | | | | | | | |
|---|---|---|---|---|---|---|---|
| A1 | Xylitol 4% | NAC 3mmol/l | Glycine 250mmol/l | | Cl₂Ca 25mmol/l | Sodium heparin 0.5 U/ml | Albumin 5% |
| B1 | Xylitol 4% | | Glycine 250mmol/l | EDTACaNa₂ 25mmol/l | Cl₂Ca 25mmol/l | Sodium heparin 0.5 U/ml | Albumin 5% |
| Cl | Xylitol 4% | NAC 3mmol/l | Glycine 250mmol/l | EDTACaNa₂ 25mmol/l | Cl₂Ca 25mmol/l | | Albumin 5% |

gives the following stability results:

| 8 weeks at 25°C | Recovery of FVIII activity (%) | Recovery of vWF:RCo activity (%) |
|---|---|---|
| A1 | 61.8 | 41.1 |
| B1 | 62.5 | 66.2 |
| C1 | 74.2 | 19.8 |

These results indicate that the protective effect on FVIII:C activity of a powerful reducing agent, such as NAC, can be substituted by the addition of a chelating agent for metals. The presence of NAC brings about a greater loss of vWF activity. Heparin is observed to act as a protector of vWF activity.

With formulation B1, at an FVIII concentration of 25 IU/ml, and which includes EDTA and sodium heparin, which is stable for 8 weeks at 25°C, it is possible to extrapolate a shelf life of at least 8 months at 5°C, thanks to the results of Example 1.

### Example 3:

An FVIII/vWF complex (25 IU FVIII/ml) formulated with:

| | | | | | | | |
|---|---|---|---|---|---|---|---|
| A2 | NAC 5 mmol/l | Glycine 250mmol/l | Arginine 150mmol/l | EDTACaNa₂ 25mmol/l | Cl₂Ca 25 mmol/l | Sodium heparin 0.5 U/ml | Albumin 5% |
| B2 | Vitamin C | Glycine 250mmol/l | Arginine 150mmol/l | EDTACaNa₂ 25mmol/l | Cl₂Ca 25 mmol/l | Sodium heparin 0.5 U/ml | Albumin 5% |
| C2 | Vitamin E | Glycine 250mmol/l | Arginine 150mmol/l | EDTACaNa₂ 25mmol/l | Cl₂Ca 25 mmol/l | Sodium heparin 0.5 U/ml | Albumin 5% |
| D2 | Vitamin C+E | Glycine 250mmol/l | Arginine 150mmol/l | EDTACaNa₂ 25mmol/l | Cl₂Ca 25 mmol/l | Sodium heparin 0.5 U/ml | Albumin 5% |

gives the following stability results:

| 8 weeks at 25°C | Recovery of FVIII activity (%) | Recovery of vWF:RCo activity (%) |
|---|---|---|
| A2 | 70.4 | 18.3 |
| B2 | 69.6 | 62.7 |
| C2 | 69.6 | 75.2 |
| D2 | 73.2 | 65.9 |

In a solution containing a chelating agent for metals and heparin, the presence of a powerful reducing agent (NAC) brings about a loss of vWF:RCo activity, without great benefit to FVIII:C, whereas, with the formulations B2, C2 and D2, at a concentration of FVIII of 25 IU/ml including EDTA and sodium heparin, it is possible to extrapolate a shelf life of at least 8 months at 5°C.

### Example 4:

An FVIII/vWF complex (25 IU FVIII/ml) formulated with:
glycine 280 mmol/l, arginine 350 mmol/l, histidine 25
mmol/l, CaCl₂ 50 mmol/l, albumin 5%, vitamin C 100 mmol/l, Tween 80 50 ppm, pH 5.10 and a variable concentration of EDTACaNa₂ and sodium heparin:

| | EDTA Concentration | Heparin Concentration |
|---|---|---|
| A3 | High (50mmol/l) | High (1 U/ml) |
| B3 | High (50mmol/l) | Low (0.2 U/ml) |
| C3 | Low (10mmol/l) | High (1 U/ml) |
| D3 | Low (10mmol/l) | Low (0.2 U/ml) |

gives the following stability results:

| 8 weeks at 25°C | Recovery of FVIII activity (%) | Recovery of vWF:RCo activity (%) |
|---|---|---|
| A3 | 85 | 71.7 |
| B3 | 63.5 | 65.9 |
| C3 | 70.4 | 95 |
| D3 | 63.5 | 89.5 |

At an FVIII concentration of 25 IU/ml, the combined addition of heparin and a metal chelator (EDTA) provides for the formulations described above a high degree of stability, in respect of the activity of vWF. It is possible to extrapolate a shelf life of at least 8 months at 5°C.

### Example 5:

Study of the proposed formula (glycine 280 mmol/l, arginine 350 mmol/l, histidine 25mmol/l, CaCl₂ 50 mmol/l, heparin 1 U/ml, EDTACaNa₂ 50 mmol/l, albumin 5%, vitamin C 100 mmol/l, Tween 80 50 ppm, pH of formulation 6.3) at 200 IU FVIII/ml in six independent batches of product gives the following stability results (8 weeks at 25°C) :

| Weeks at 25°C | 0 | 2 | 4 | 6 | 8 |
|---|---|---|---|---|---|
| FVIII:C (%) Mean±SD (n=6) | 100 | 91.0±6.3 | 65.5±8.0 | 48.2±8.1 | 35.4±9.1 |
| vWF:RCo (%) Mean±SD (n=6) | 100 | 105.4±8.5 | 87.7±19.7 | 94.7±17.5 | 85.8±13.8 |

When the concentration of product is increased (from 25 to 200 IU FVIII/ml) lower stability is observed. The monitoring of the evolution of activity throughout the study includes, in addition to the initial and final times, sampling at 2, 4 and 6 weeks of stability. Using all of the data derived from the six batches studied, it is possible to estimate the overall behaviour of the activities of FVIII:C and vWF:RCo, fitting it to first-order kinetics. From this analysis it is possible to estimate that recoveries of the order of 50% of activity (FVIII and vWF) would be obtained after 6 weeks at 25°C, which is equivalent to six months at 5°C.

### Example 6:

Study of the protective effect of a protease inhibitor. The proposed formula was tested at 200 IU FVIII/ml on 6 independent batches of product.

Glycine 280 mmol/l, arginine 350 mmol/l, histidine 25mmol/l, CaCl₂ 50 mmol/l, albumin 5%, vitamin C 100 mmol/l, Tween 80 50 ppm, pH of formulation 6.3; heparin 1 IU/ml, EDTACaNa₂ 50 mmol/l, and antithrombin (0.05 IU/IU FVIII), giving the following stability results (8 weeks at 25°C) :

| Weeks at 25°C | 0 | 2 | 4 | 6 | 8 |
|---|---|---|---|---|---|
| FVIII:C (%) Mean±SD (n=6) | 100 | 94.5±4.5 | 86.9±5.2 | 71.5±3.2 | 67.6±5.0 |
| vWF:RCo (%) Mean±SD (n=6) | 100 | 101.5±16.7 | 117.4±28.5 | 108.1±22.3 | 91.1±24.0 |

The addition of a protease inhibitor (antithrombin) increases the stability of the product both in relation to Factor VIII and for the activity of ristocetin cofactor (vWF). An analysis parallel with that carried out in Example 5 makes it possible to estimate 50% recoveries of activity (FVIII and vWF:RCo) after 12 weeks at 25°C, which is equivalent to 12 months at 5°C.

It will be appreciated from the above Examples that, at a low concentration of product (25 IU FVIII/ml), the addition of a chelating agent for metals (EDTA) and heparin provides greater stability for the FVIII/vWF complex. This stabilisation improves with the addition of a protease inhibitor (antithrombin) even at high concentrations of FVIII and vWF activity.

## Claims

1. Biologically stable liquid composition of FVIII or of vWF or of FVIII/vWF complex of human origin, **characterised in that** it comprises a concentrate of FVIII or of vWF or of FVIII/vWF complex of human origin with a specific ligand selected from heparin and sodium heparin, a chelating agent for metals, and preferably a protease inhibitor.

2. Biologically stable liquid composition of FVIII or of vWF or of FVIII/vWF complex of human origin according to claim 1, **characterised in that** the chelating agent for metals is used at a concentration of 2-100 mmol/l of the concentrate.

3. Biologically stable liquid composition of FVIII or of vWF or of FVIII/vWF complex of human origin according to claim 1, **characterised in that** the chelating agent for metals is EDTA.

4. Biologically stable liquid composition of FVIII or of vWF or of FVIII/vWF complex of human origin according to claim 1, **characterised in that** the chelating agent for metals is EDTACaNa₂ (sodium and calcium salt of EDTA).

5. Biologically stable liquid composition of FVIII or of vWF or of FVIII/vWF complex of human origin, according to claim 1, **characterised in that** the ligand is included at a concentration of 0.1-10 U/ml of the concentrate.

6. Biologically stable liquid composition of FVIII or of vWF or of FVIII/vWF complex of human origin according to claim 1, **characterised in that** the ligand is sodium heparin.

7. Biologically stable liquid composition of FVIII or of vWF or of FVIII/vWF complex of human origin according to claim 1, **characterised in that** the protease inhibitor is present at a concentration greater than 0.01 IU/IU FVIII.

8. Biologically stable liquid composition of FVIII or of vWF or of FVIII/vWF complex of human origin according to claim 7, **characterised in that** the protease inhibitor is present at a concentration of 0.01-1 IU/IU FVIII.

9. Biologically stable liquid composition of FVIII or of vWF or of FVIII/vWF complex of human origin according to claim 1, **characterised in that** the protease inhibitor is a serine protease inhibitor (serpin).

10. Biologically stable liquid composition of FVIII or of vWF or of FVIII/vWF complex of human origin according to claim 9, **characterised in that** the serine protease inhibitor is antithrombin.

## Patentansprüche

1. Biologisch stabile flüssige Zusammensetzung von FVIII oder von vWF oder eines FVIII/vWF-Komplexes menschlicher Herkunft, **dadurch gekennzeichnet, dass** sie ein Konzentrat von FVIII oder von vWF oder eines FVIII/vWF-Komplexes menschlicher Herkunft mit einem spezifischen Liganden, der aus Heparin und Natriumheparin ausgewählt ist, einen Chelatbildner für Metalle, und bevorzugt einen Protease-Inhibitor, aufweist.

2. Biologisch stabile flüssige Zusammensetzung von FVIII oder von vWF oder eines FVIII/vWF-Komplexes menschlicher Herkunft nach Anspruch 1, **dadurch gekennzeichnet, dass** der Chelatbildner für Metalle in einer Konzentration von 2 bis 100 mmol/l des Konzentrats verwendet wird.

3. Biologisch stabile flüssige Zusammensetzung von FVIII oder von vWF oder eines FVIII/vWF-Komplexes menschlicher Herkunft nach Anspruch 1, **dadurch gekennzeichnet, dass** der Chelatbildner für Metalle EDTA ist.

4. Biologisch stabile flüssige Zusammensetzung von FVIII oder von vWF oder eines FVIII/vWF-Komplexes menschlicher Herkunft nach Anspruch 1, **dadurch gekennzeichnet, dass** der Chelatbildner für Metalle EDTACaNa₂ (Natrium- und Calcium-Salz von EDTA) ist.

5. Biologisch stabile flüssige Zusammensetzung von FVIII oder von vWF oder eines FVIII/vWF-Komplexes menschlicher Herkunft nach Anspruch 1, **dadurch gekennzeichnet, dass** der Ligand in einer Konzentration von 0.1- 10 U/ml des Konzentrats enthalten ist.

6. Biologisch stabile flüssige Zusammensetzung von FVIII oder von vWF oder eines FVIII/vWF-Komplexes menschlicher Herkunft nach Anspruch 1, **dadurch gekennzeichnet, dass** der Ligand Natriumheparin ist.

7. Biologisch stabile flüssige Zusammensetzung von FVIII oder von vWF oder eines FVIII/vWF-Komplexes menschlicher Herkunft nach Anspruch 1, **dadurch gekennzeichnet, dass** der Protease-Inhibitor in einer Konzentration von größer als 0,01 IU/IU FVIII vorliegt.

8. Biologisch stabile flüssige Zusammensetzung von FVIII oder von vWF oder eines FVIII/vWF-Komplexes menschlicher Herkunft nach Anspruch 7, **dadurch gekennzeichnet, dass** der Protease-Inhibitor in einer Konzentration von 0,01 bis 1 IU/IU FVIII vorliegt.

9. Biologisch stabile flüssige Zusammensetzung von FVIII oder von vWF oder eines FVIII/vWF-Komplexes menschlicher Herkunft nach Anspruch 1, **dadurch gekennzeichnet, dass** der Protease-Inhibitor ein Serin-Protease-Inhibitor (Serpin) ist.

10. Biologisch stabile flüssige Zusammensetzung von FVIII oder von vWF oder eines FVIII/vWF-Komplexes menschlicher Herkunft nach Anspruch 9, **dadurch gekennzeichnet, dass** der Serin-Protease-Inhibitor Antithrombin ist.

## Revendications

1. Composition liquide biologiquement stable de FVIII, de vWF ou d'un complexe FVIII/vWF d'origine humaine, **caractérisée en ce qu'**elle comprend un concentré de FVIII, de vWF ou d'un complexe FVIII/vWF d'origine humaine avec un ligand spécifique choisi parmi l'héparine et l'héparine sodique, un agent chélatant pour métaux et de préférence un inhibiteur de protéase.

2. Composition liquide biologiquement stable de FVIII, de vWF ou d'un complexe FVIII/vWF d'origine humaine selon la revendication 1, **caractérisée en ce que** l'agent chélatant pour métaux est utilisé à une concentration de 2 à 100 mmol/l de concentré.

3. Composition liquide biologiquement stable de FVIII, de vWF ou d'un complexe FVIII/vWF d'origine humaine selon la revendication 1, **caractérisée en ce que** l'agent chélatant pour métaux est de l'EDTA.

4. Composition liquide biologiquement stable de FVIII, de vWF ou d'un complexe FVIII/vWF d'origine humaine selon la revendication 1, **caractérisée en ce que** l'agent chélatant pour métaux est de l'EDTACaNa₂.

5. Composition liquide biologiquement stable de FVIII, de vWF ou d'un complexe FVIII/vWF d'origine humaine, selon la revendication 1, **caractérisée en ce que** le ligand est inclus à une concentration de 0,1 à 10 U/ml de concentré.

6. Composition liquide biologiquement stable de FVIII, de vWF ou d'un complexe FVIII/vWF d'origine humaine selon la revendication 1, **caractérisée en ce que** le ligand est de l'héparine sodique.

7. Composition liquide biologiquement stable de FVIII, de vWF ou d'un complexe FVIII/vWF d'origine humaine selon la revendication 1, **caractérisée en ce que** l'inhibiteur de protéase est présent à une concentration supérieure à 0,01 IU/IU de FVIII.

8. Composition liquide biologiquement stable de FVIII, de vWF ou d'un complexe FVIII/vWF d'origine humaine selon la revendication 7, **caractérisée en ce que** l'inhibiteur de protéase est présent à une concentration de 0,01 à 1 IU/IU de FVIII.

9. Composition liquide biologiquement stable de FVIII, de vWF ou d'un complexe FVIII/vWF d'origine humaine selon la revendication 1, **caractérisée en ce que** l'inhibiteur de protéase est un inhibiteur de sérine protéase (serpine).

10. Composition liquide biologiquement stable de FVIII, de vWF ou d'un complexe FVIII/vWF d'origine humaine selon la revendication 9, **caractérisée en ce que** l'inhibiteur de sérine protéase est l'antithrombine.
